# DEMANDE DE BREVET EUROPEEN

(11) **EP 1 656 968 A1**
(43) Date de publication de la demande: **17.05.2006**
(21) Numéro de dépôt: 05300927.0
(22) Date de dépôt: 15.11.2005
(51) Int. Cl.: A61Q 5/00, B65D 83/14

(54) **Dispositif pressurisé pour le traitement des fibres capillaires**

(30) Priorité: 16.11.2004 FR 0452646
(71) Demandeur: L'OREAL, 75008 Paris (FR)
(72) Inventeur: Jourdan, Hervé, 78210 Saint-Cyr (FR); Beitone, Régis, 75013 Paris (FR); Le Bourhis, François, 93300 Aubervilliers (FR); Lasserre, Pierre-André, 93470 Coubron (FR)
(74) Mandataire: Michelet, Alain

(57) **Abrégé**

La présente invention se rapporte à un dispositif pressurisé pour le coiffage des fibres capillaires et à son utilisation pour la mise en forme et/ou le soin des fibres capillaires. Le dispositif pressurisé, notamment aérosol, comprend un récipient dont la paroi est formée au moins en partie d'un mélange d'au moins un polyéthylène naphtalate et d'au moins un polyéthylène téréphtalate, une composition cosmétique comprenant une phase aqueuse comprenant au moins un agent moussant ; et une phase organique comprenant au moins un agent propulseur choisi parmi les hydrocarbures halogénés ou non et leurs mélanges, ledit dispositif étant résistant à la température.

## Description

La présente invention se rapporte à un dispositif pressurisé comprenant une composition cosmétique et à son utilisation pour la mise en forme et/ou le soin des fibres capillaires.

Les compositions cosmétiques conditionnées dans des dispositifs aérosols sont habituellement des compositions à pulvériser, pressurisées à l'aide d'un agent propulseur dans des récipients métalliques, en aluminium ou en fer blanc.

De tels dispositifs pressurisés sont souvent stockés avant commercialisation dans des entrepôts où la durée de stockage peut aller jusqu'à plusieurs mois.

Stockés dans de telles conditions, ces dispositifs pressurisés peuvent être soumis à de fortes températures.

Or ces dispositifs pressurisés, surtout lorsqu'ils sont en matière plastique, présentent l'inconvénient de ne pas résister à la température, ce qui peut entraîner leur déformation, voire leur éclatement.

Le but de l'invention est donc de fournir un dispositif pressurisé, notamment aérosol comprenant une composition cosmétique, résistant à la température et qui n'éclate pas ou ne se déforme pas, notamment lors de cycles de stockage longs.

La demanderesse a donc trouvé de façon surprenante qu'il était possible de remédier aux inconvénients de l'art antérieur et de remplir les objectifs précités en réalisant un dispositif pressurisé, notamment aérosol comprenant un récipient dont la paroi est formée d'au moins un polyéthylène naphtalate, une composition cosmétique aqueuse biphasée comprenant au moins un agent propulseur choisi parmi les hydrocarbures et leurs mélanges, et au moins un agent moussant, ladite composition étant restituée au moyen d'un diffuseur sous forme de mousse.

Ainsi, l'invention a pour objet un dispositif pressurisé, notamment aérosol, comprenant :
a) un récipient dont la paroi est formée au moins en partie d'un mélange d'au moins un polyéthylène naphtalate et d'au moins un polyéthylène téréphtalate,
b) une composition cosmétique comprenant :
   - une phase aqueuse comprenant au moins un agent moussant ; et
   - une phase organique comprenant au moins un agent propulseur choisi parmi les hydrocarbures halogénés ou non et leurs mélanges,
ledit dispositif étant résistant à la température.

On entend par dispositif résistant à la température au sens de la présente invention un dispositif qui, porté à une température de 80°C et maintenu à une telle température pendant deux heures, n'explose pas.

De préférence, le récipient selon l'invention comprend, en poids par rapport au poids total de polymère, de 60% à 95%, plus préférentiellement 90% de polyéthylène naphtalate et de 5% à 40%, plus préférentiellement 10% de polyéthylène téréphtalate.

De préférence, le récipient du dispositif selon l'invention est transparent ou translucide.

On entend par récipients transparents ou translucides au sens de la présente invention des récipients au travers de la paroi desquels on peut voir la composition cosmétique.

De préférence, la composition comprise dans le dispositif selon l'invention est transparente ou translucide.

On entend par compositions transparentes ou translucides au sens de la présente invention des compositions présentant une turbidité, mesurée selon la méthode décrite ci-après, inférieure à 800 NTU (Nephelometric Turbidity Units) et de préférence inférieure à 500 NTU.

On mesure la turbidité à l'aide d'un turbidimètre modèle 2100 P de la société HACH™ à température ambiante (20 à 25°C). Les tubes utilisés pour la mesure sont référencés AR 397 A cat 24347-06. L'étalonnage de l'appareil se fait par des suspensions de formazine de différentes concentrations.

On entend par mousse au sens de la présente invention un mélange comprenant la composition cosmétique et l'agent propulseur, et dont la densité par rapport à l'eau, mesurée à la température de 25°C et à la pression atmosphérique (1,013 bar), peut aller jusqu'à 0,1.

Au sens de la présente invention, on entend par composition biphasique une composition comprenant une phase aqueuse et une phase organique, non miscibles l'une avec l'autre.

De préférence, au moins une des deux phases de la composition cosmétique est transparente.

Lorsque le récipient selon l'invention est constitué d'un mélange de polyéthylène naphtalate et de polyéthylène téréphtalate, ce mélange comprend au moins 50%, de préférence au moins 75% en poids de polyéthylène naphtalate par rapport au poids total du mélange de polymères.

Comme indiqué précédemment, la composition selon l'invention comprend au moins un agent moussant choisi parmi les tensio-actifs cationiques, anioniques, non ioniques, amphotères ou zwittérioniques et les polymères cationiques, anioniques, non ioniques, amphotères ou zwittérioniques.

Au sens de la présente invention, on entend par agent moussant un agent qui, en présence d'un mélange d'eau, d'agent propulseur et du dispositif de restitution adéquat forme une mousse.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante:

R₁(̵OC₂H₄)̵ₙ OCH₂COOA (1)

dans laquelle :
R₁ désigne un groupement alkyle alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPOS (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10 - C14) amines ou les oxydes de N-acylaminopropylmorpholine.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C8-C20) bétaïnes, les sulfobétaïnes, les alkyl (C8-C20) amidoalkyl (C1-C6) betaïnes ou les alkyl (C8-C20) amidoalkyl (C1-C6) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH2CH2 -N+(R₃)(R₄)(CH2COO-) (2)

dans laquelle : R₂ désigne un radical alkyle d'un acide R2-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH2CH2-N(B)(C) (3)

dans laquelle :
B représente -CH2CH2OX', C représente -(CH2)z -Y', avec z = 1 ou 2,
X' désigne le groupement -CH2CH2-COOH ou un atome d'hydrogène
Y' désigne -COOH ou le radical -CH2 - CHOH - SO3H
R₂' désigne un radical alkyle d'un acide R9 -COOH présent dans l'huile de coprah ou dans l'huile de lin hydrolysée, un radical alkyle, notamment en C7, C9, C11 ou C13, un radical alkyle en C17 et sa forme iso, un radical C17 insaturé.

A titre d'exemple on peut citer le cocoamphocarboxyglycinate vendu sous la dénomination commerciale MIRANOL C2M concentré par la Société MIRANOL.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

On peut également utiliser pour la présente invention tout polymère connu dans le domaine des traitements capillaires ayant des propriétés moussantes, ainsi que des mélanges contenant plusieurs de ces polymères. On distingue classiquement les polymères cationiques, anioniques, non ioniques, amphotères ou zwittérioniques.

Les polymères cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs suivants : 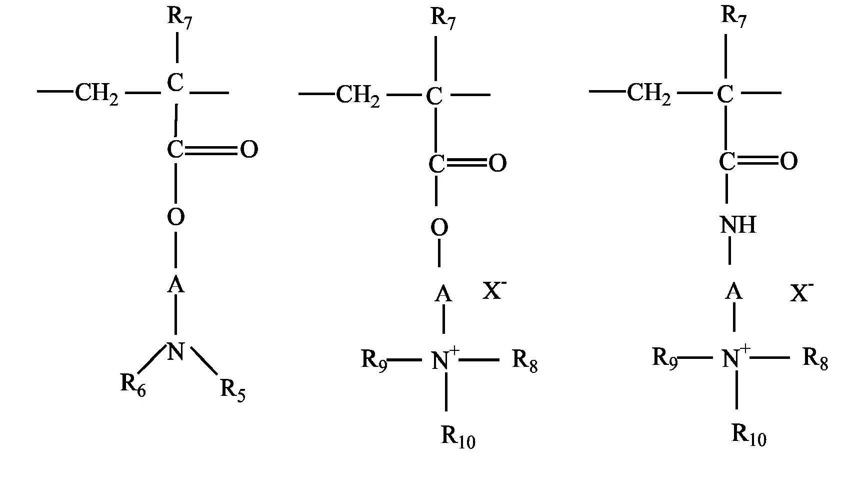 dans lesquels :
   R₅ et R₆ représentent chacun un atome d'hydrogène ou un groupe alkyle en C₁₋₆,
   R₇ désigne un atome d'hydrogène ou un radical CH₃ .
   R₈, R₉ et R₁₀, identiques ou différents, représentent chacun un groupe alkyle en C₁₋₁₈ ou un radical benzyle, A est un groupe alkyle linéaire ou ramifié en C₁₋₆ ou un groupe hydroxyalkyle en C₁₋₄, et
   X⁻ désigne un anion méthosulfate ou halogénure tel qu'un ion chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieurs, des acides acryliques ou méthacryliques ou leurs esters, des vinyl lactames tels que la vinylpyrrolidone ou le vinylcaprolactame, et des esters vinyliques.
   Les motifs vinyl lactames préférés selon l'invention sont ceux qui comprennent des motifs de formule : 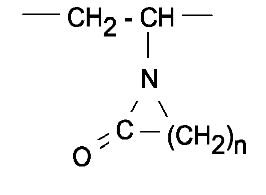 dans laquelle n est indépendamment 3, 4 ou 5.
   De manière avantageuse, les polymères à motifs vinyl lactames sont des copolymères ou peuvent également comprendre des motifs de formule 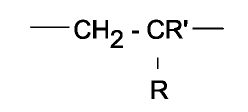 dans laquelle
   R représente indépendamment un radical carboxy, un radical carbalcoxy, un radical acyloxy, le groupement alkoxy du radical carbalcoxy pouvant être substitué par au moins un radical hydroxy, amino, alkylamino ou dialkylamino, ou un radical aryle, en particulier phényle, éventuellement substitué par au moins un radical alkyle, et
   R' représente un atome d'hydrogène ou un radical alkyle.
   Par alkyle, on entend de préférence selon l'invention les radicaux alyles linéaires ou ramifiés en C₁-C₁₀, plus préférentiellement les radicaux alkyles en C₁-C₄, en particulier les radicaux méthyle, éthyle, n-propyle, i-propyle, n-butyle et t-butyle.
   Par acyle, on entend de préférence selon l'invention les radicaux acyles dont le reste alkyle est un alkyle linéaire ou ramifié en C₁-C₁₀, en particulier les radicaux acétyle ou propionyle.
   Les polymères à motifs vinyl lactames selon l'invention sont de manière préférentielle des polymères non ioniques ou faiblement cationiques. Ils sont notamment décrits dans les brevets US 3 770 683, US 3 929 735, US 4 521 504, US 5 158 762, US 5 506 315 et dans les demandes de brevet WO 94/121148, WO 96/06592 et WO 96/10593.
   Ainsi, on peut citer parmi les copolymères de la famille (1) :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la Société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT® P 100 par la Société CIBA GEIGY,
   - le copolymère d'acrylamide et de méthosulfate de méthacryloyloxyéthyltriméthylammonium vendu sous la dénomination RETEN® par la Société HERCULES,
   - les copolymères vinylpyrrolidone/acrylate ou méthacrylate de dialkylaminoalkyle quaternisés ou non, tels que les produits vendus sous la dénomination GAFQUAT® par la Société ISP, comme par exemple GAF-QUAT® 734 OU GAFQUAT® 755, ou bien les copolymères polyvinylpyrrolidone/méthacrylatede diaminoéthyle non quaternisé dénommés COPOLYMER® 845 , 958 et 937. Ces polymères sont décrits en détail dans les demandes de brevets français FR 2 077 143 et FR 2 393 573,
   - les terpolymères méthacrylate de diméthyl-aminoéthyle/vinylcaprolactame/vinylpyrrolidone tels que le produit commercialisé sous la dénomination GAFFIX VC 713 par la Société ISP, et
   - le copolymère vinylpyrrolidone/méthacrylamide de diméthylaminopropyle quaternisé tel que le produit vendu sous la dénomination GAFQUAT® HS 100 par la Société ISP.
(2) les polysaccharides quaternisés décrits plus particulièrement par les brevets américains 3,589,578 et 4,031,307 tels que les gommes de guar contenant des groupements cationiques trialkylammonium.
   De tels produits sont commercialisés notamment sous les dénominations commerciales de JAGUAR@ C13 S, JAGUAR@ C15 et JAGUAR@ C17 par la Société MEYHALL.
(3) Les copolymères quaternaires de vinylpyrrolidone et de vinylimidazole tels que les produits commercialisés par BASF sous la dénomination LUVIQUAT® TFC,
(4) les chitosanes ou leurs sels, en particulier les acétate, lactate, glutamate, gluconate ou pyrrolidonecarboxylate de chitosane.
   On peut citer le chitosane ayant un taux de désacétylation de 90,5% en poids vendu sous la dénomination KYTAN BRUT STANDARD® par la Société ABER TECHNOLOGIES, le pyrrolidonecarboxylate de chitosane vendu sous la dénomination KYTAMER® PC par la Société AMERCHOL.
(5) Les dérivés de cellulose cationiques tels que les copolymères de cellulose ou les dérivés de cellulose greffés avec un monomère hydrosoluble comportant un groupe ammonium quaternaire et décrits notamment dans le brevet US 4,131,576 tels que les hydroxyalkylcelluloses, comme les hydroxyméthylhydroxyéthyl- ou hydroxypropylcelluloses greffées notamment avec un sel de méthacryloyloxyéthyltriméthylammonium, de méthacrylamidopropyltriméthylammonium ou de diméthyldiallylammonium.

Les produits commercialisés répondant à cette définition sont plus particulièrement les produits vendus sous les dénominations CELQUAT® L 200 et CELQUAT® H 100 par la Société NATIONAL STARCH.

Les polymères anioniques généralement utilisés sont des polymères comportant des groupements dérivés d'un acide carboxylique, sulfonique ou phosphorique et ont un poids moléculaire moyen en poids compris entre environ 500 et 5 000 000.

Les groupements acide carboxyliques sont apportés par des monomères insaturés contenant une ou deux fonctions acide carboxylique, tels que ceux répondant à la formule (4) : 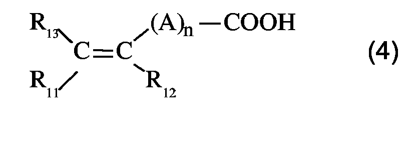 dans laquelle n est un nombre entier de 0 à 10, A désigne un groupement méthylène, éventuellement relié à l'atome de carbone du groupement insaturé ou du groupement méthylène voisin, lorsque n est supérieur à 1, par l'intermédiaire d'un hétéroatome tel que oxygène ou soufre, R₁₃ désigne un atome d'hydrogène, un groupement phényle ou benzyle, R₁₁ désigne un atome d'hydrogène, un groupement alkyle inférieur ou carboxyle, et R₁₂ désigne un atome d'hydrogène, un groupement alkyle inférieur, un groupement -CH₂-COOH, phényle ou benzyle.

Dans la formule précitée un radical alkyle inférieur désigne de préférence un groupement ayant de 1 à 4 atomes de carbone et en particulier un groupe méthyle ou éthyle.

Les polymères anioniques carboxylés préférés selon l'invention sont :
A) les homo- ou copolymères d'acide acrylique ou méthacrylique ou leurs sels et en particulier les produits commercialisés sous les dénominations VERSICOL® E ou K par la Société ALLIED COLLOID, et sous la dénomination ULTRAHOLD® par la Société BASF ; les copolymères d'acide acrylique et d'acrylamide commercialisés sous forme de sel de sodium sous les dénominations RETEN® 421, 423 ou 425 par la Société HERCULES ; les sels de sodium des acides polyhydroxycarboxyliques.
B) Les copolymères d'acide acrylique ou d'acide méthacrylique et d'un monomère monoéthylénique tel que l'éthylène, le styrène, les esters vinyliques, les esters d'acide acrylique ou méthacrylique.
   Ces copolymères peuvent être greffés sur un polyalkylène glycol tel que le polyéthylèneglycol et sont éventuellement réticulés.
   De tels polymères sont décrits en particulier dans la demande de brevet français FR 1 222 944 et dans la demande de brevet allemand DE 2 330 956. On peut notamment citer les copolymères comportant dans leur chaîne un motif acrylamide éventuellement N-alkylé et/ou hydroxyalkylé, tels que ceux décrits dans les demandes de brevet luxembourgeois LU 75370 et LU75371 ou proposés sous la dénomination QUADRAMER® par la Société AMERICAN CYANAMID.
   On peut également citer les copolymères d'acide acrylique et de méthacrylate d'alkyle en C₁-C₄, et les terpolymères de vinylpyrrolidone, d'acide (méth)acrylique et de (méth)acrylate d'alkyle en C₁-C₂₀, par exemple de lauryle (ACRYLDONE® LM de la Société ISP), de tertiobutyle (LUVIFLEX® VBM 70 commercialisé par BASF) ou de méthyle (STEPANHOLD® EXTRA commercialisé par STEPAN), et les terpolymères acide méthacrylique/acrylate d'éthyle/acrylate de tertiobutyle tel que le produit commercialisé sous la dénomination LUVIMER® 100 P par la Société BASF, et les terpolymères polyvinyl pyrrolidone / acétate de vinyle / propionate de vinyle, en particulier ceux commercialisés sous la dénomination Luviskol VAP 343 par la société BASF.
C) Les copolymères dérivés d'acide crotonique tels que ceux comportant dans leur chaîne des motifs acétate ou propionate de vinyle et éventuellement d'autres monomères tels que les esters allylique, méthallylique ou vinylique d'un acide carboxylique saturé, linéaire ou ramifié, à longue chaîne hydrocarbonée comportant au moins 5 atomes de carbone, ces polymères pouvant éventuellement être greffés et réticulés, ou encore les esters vinylique, allylique ou méthallylique d'un acide carboxylique α- ou β-cyclique.
   De tels polymères sont décrits, entre autres, dans les demandes de brevets français FR 1 222 944, FR 1 580 545, FR 2 265 782, FR 2 265 781, FR 1 564 110 et FR 2 439 798.
   On peut citer à titre d'exemples de produits commerciaux entrant dans cette classe les résines 28-29-30, 26-13-14 et 28-13-10 commercialisées par la Société NATIONAL STARCH.
D) Les copolymères dérivés d'acides ou d'anhydrides carboxyliques monoinsaturés en C₄- C₈ choisis parmi :
   - les copolymères comprenant :
      (i) un ou plusieurs acides ou anhydrides maléique, fumarique, itaconique et
      (ii) au moins un monomère choisi parmi les esters vinyliques, les éthers vinyliques, les halogénures vinyliques, les dérivés phénylvinyliques, l'acide acrylique et ses esters, les fonctions anhydride de ces copolymères étant éventuellement monoestérifiées ou monoamidifiées.
         De tels polymères sont décrits en particulier dans les demandes de brevets US 2,047,398, US 2,723,248, US 2,102,113 et GB 839,805 et notamment ceux commercialisés sous les dénominations GANTREZ AN ou ES, AVANTAGE® CP par la Société ISP ;
   - les copolymères comprenant
      (iii) un ou plusieurs anhydrides maléique, citraconique ou itaconique et
      (iv) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
      Ces polymères sont par exemple décrits dans les demandes de brevets français FR 2 350 384 et FR 2 357 241.
E) Les polyacrylamides comportant des groupements carboxylate.
   Les groupements anioniques des polymères anioniques de la présente invention peuvent également être des groupes acide sulfonique apportés par des motifs vinylsulfonique, styrènesulfonique, naphtalènesulfonique ou acrylamidoalkylsulfonique.
   Ces polymères à groupes acide sulfonique sont notamment choisis parmi :
   - les sels de poly(acide vinylsulfonique) ayant un poids moléculaire moyen en poids compris entre environ 1000 et 100 000 ainsi que les copolymères d'acide vinylsulfonique et d'un comonomère insaturé tel que l'acide acrylique, l'acide méthacrylique, les esters de ces acides, l'acrylamide, les dérivés d'acrylamide, les éthers vinyliques et la vinylpyrrolidone ;
   - les sels de poly(acide styrènesulfonique). On peut citer à titre d'exemple deux sels de sodium ayant un poids moléculaire moyen en poids d'environ 500 000 et d'environ 100 000 commercialisés respectivement sous les dénominations FLEXAN® 500 et FLEXAN® 130 par la Société NATIONAL STARCH. Ces composés sont décrits dans le brevet FR 2 198 719 ;
   - les sels de poly(acide acrylamidesulfonique), tels que ceux mentionnés dans le brevet US 4,128,631 et plus particulièrement le poly(acide acrylamidoéthyl-propanesulfonique) commercialisé sous la dénomination COSMEDIA POLYMER® HSP 1180 par la Société HENKEL.

Selon l'invention, les polymères anioniques sont de préférence choisis parmi les copolymères d'acide acrylique tels que les terpolymères acide acrylique/acrylate d'éthyle/N-tertiobutylacrylamide commercialisés notamment sous la dénomination ULTRAHOLD STRONG® par la Société BASF, les copolymères dérivés d'acide crotonique tels que les terpolymères acétate de vinyle/tertio-butyl benzoate de vinyle/acide crotonique et les terpolymères acide crotonique/acétate de vinyle/néododécanoate de vinyle commercialisés notamment sous la dénomination Résine 28-29-30 par la Société NATIONAL STARCH, les copolymères dérivés d'acide ou d'anhydride maléique, fumarique ou itaconique et contenant comme comonomères des esters vinyliques, des éthers vinyliques, des halogénures vinyliques, des dérivés phénylvinyliques, l'acide acrylique et les esters d'acide acrylique, tels que les copolymères méthylvinyléther/anhydride maléique monoestérifié, commercialisés par exemple sous la dénomination GANTREZ® par la Société ISP, les copolymères d'acide méthacrylate de méthyle commercialisés sous la dénomination EUDRAGIT®L par la Société ROHM PHARMA, les copolymères acide méthacrylique/méthacrylate de méthyle/acrylate d'alkyle en C₁₋₄/acide acrylique ou méthacrylate d'hydroxyalkyle en C₁₋₄, commercialisés sous la dénomination AMERHOLD® DR 25 par la Société AMERCHOL, ou sous la dénomination ACUDYNE® 255 par la Société ROHM & HAAS, les copolymères d'acide méthacrylique et d'acrylate d'éthyle commercialisés sous la dénomination LUVIMER® MAEX ou MAE par la Société BASF et les copolymères acétate de vinyle/acide crotonique et les copolymères acétate de vinyle/acide crotonique greffés par du polyéthylèneglycol commercialisés sous la dénomination ARISTOFLEX® A par la Société BASF.

Les polymères amphotères utilisables pour la présente invention sont choisis notamment parmi les polymères comportant des motifs B et C répartis statistiquement dans la chaîne polymère, où B désigne un motif dérivant d'un monomère comportant au moins un atome d'azote basique et C désigne un motif dérivant d'un monomère comportant un ou plusieurs groupements acide carboxylique ou acide sulfonique. Les polymères amphotères peuvent également comporter des motifs zwittérioniques de type carboxybétaïne ou sulfobétaïne. Il peut également s'agir de polymères à chaîne principale cationique comportant des groupements amine primaire, secondaire, tertiaire ou quaternaire, parmi lesquels au moins un, porte, par l'intermédiaire d'un radical hydrocarboné, un groupement acide carboxylique ou acide sulfonique. Les polymères amphotères peuvent encore avoir une chaîne anionique dérivée d'acides dicarboxyliques α,β-insaturés dont l'un des groupements carbonyle a été amené à réagir avec une polyamine comportant un ou plusieurs groupements amine primaire ou secondaire.

Les polymères amphotères répondant à la définition donnée ci-dessus sont choisis en particulier parmi les polymères suivants :
(1) Les polymères résultant de la copolymérisation d'un monomère vinylique portant un groupement acide carboxylique tel que l'acide acrylique, l'acide méthacrylique, l'acide maléique, l'acide α-chloroacrylique, et d'un monomère vinylique contenant au moins une fonction basique tel que les méthacrylate et acrylate de dialkylaminoalkyle ou les dialkylaminoalkyl(méth)acrylamides. De tels composés sont décrits par exemple dans la demande de brevet américain US 3,836,537.
(2) Les polymères comportant des motifs dérivés :
   (a) d'au moins un monomère choisi parmi les acrylamides ou méthacrylamides N-alkylés,
   (b) d'au moins un comonomère contenant une ou plusieurs fonctions acide carboxylique, et
   (c) d'au moins un comonomère basique tel que des esters à substituants amine primaire, secondaire, tertiaire ou quaternaire d'acide acrylique et d'acide méthacrylique, et le produit de quaternisation du méthacrylate de diméthylaminoéthyle avec le sulfate de diméthyle ou diéthyle.
      Les acrylamides ou méthacrylamides N-alkylés (a) préférés sont ceux portant des radicaux alkyle en C₂₋₁₂, tels que le N-éthylacrylamide, le N-tertiobutylacrylamide, le N-tertio-octylacrylamide, le N-octylacrylamide, le N-décylacrylamide, le N-dodécylacrylamide ainsi que les méthacrylamides correspondants.
      Les comonomères à groupe acide carboxylique (b) sont choisis plus particulièrement parmi les acides acrylique, méthacrylique, crotonique, itaconique, maléique, fumarique ainsi que parmi les monoesters d'alkyle en C₁₋₄ des acides ou des anhydrides maléique ou fumarique.
      Les comonomères basiques (c) préférés sont le méthacrylate d'aminoéthyle, le méthacrylate de butylaminoéthyle, le méthacrylate de N,N'-diméthylaminoéthyle et le méthacrylate de N-tertiobutylaminoéthyle.
      On utilise en particulier les copolymères dont la dénomination CTFA (4^{ième} Ed., 1991) est « Octylacrylamide/acrylates/butylaminoéthyl-méth-acrylate copolymer », tels que les produits commercialisés sous la dénomination AMPHOMER® ou LOVOCRYL® 47 par la Société NATIONAL STARCH.
(3) Les polyaminoamides réticulés et alcoylés, dérivés en partie ou en totalité de polyaminoamides de formule générale (5) :

   (5) -[C(=0)-R₁₄-C(=0)-Z-]-

   dans laquelle R₁₄ représente un radical divalent dérivé d'un acide dicarboxylique saturé, d'un acide aliphatique mono- ou dicarboxylique à double liaison éthylénique, d'un ester d'alkyle en C₁₋₆ de ces acides ou d'un radical dérivant de l'addition de l'un quelconque desdits acides sur une amine bis primaire ou bis secondaire, et Z désigne un radical d'une polyalkylène-polyamine bis-primaire, mono- ou bis-secondaire, et de préférence représente :
   a) dans les proportions de 60 à 100 % en moles, le radical de formule (6) :

      (6) -NH-[(CH₂)ₓ-NH]ₚ-

      où x = 1 et p = 2 ou 3, ou bien x = 3 et p = 2,
      ce radical dérivant de la diéthylènetriamine, de la triéthylènetétraamine
      ou de la dipropylènetriamine ;
   b) dans les proportions de 0 à 40% en moles le radical de formule (III) dans lequel x = 2 et p = 1, dérivé de l'éthylènediamine, ou le radical 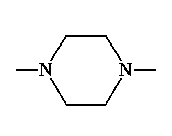 dérivé de la pipérazine :
   c) dans les proportions de 0 à 20 % en moles le radical -NH-(CH₂)₆-NH- dérivé de l'hexaméthylènediamine, ces polyaminoamines étant réticulées par addition de 0,025 à 0,35 mole par mole de groupement amine, d'un agent réticulant bifonctionnel choisi parmi les épihalohydrines, les diépoxydes, les dianhydrides, les composés di-insaturés, et alcoylés par l'acide acrylique, l'acide chloracétique ou une alcane-sulfone.
      Les acides carboxyliques saturés sont choisis de préférence parmi les acides ayant 6 à 10 atomes de carbone tels que l'acide adipique, l'acide triméthyl-2,2-4-adipique et triméthyl-2,4,4-adipique, l'acide téréphtalique, les acides à double liaison éthylénique comme par exemple les acides acrylique, méthacrylique, itaconique.
      Les alcane-sulfones utilisées dans l'alcoylation sont de préférence la propanesulfone ou la butanesulfone.
      Les sels des agents d'alcoylation sont de préférence les sels de sodium ou de potassium.
(4) Les polymères comportant des motifs zwittérionique de formule (7) : 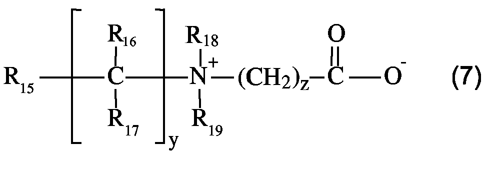 dans laquelle R₁₅ désigne un groupement insaturé polymérisable tel qu'un groupement acrylate, méthacrylate, acrylamide ou méthacrylamide, y et z représentent chacun un nombre entier de 1 à 3, R₁₆ et R₁₇ représentent chacun indépendamment un atome d'hydrogène ou un groupe méthyle, éthyle ou propyle, R₁₈ et R₁₉ représentent chacun indépendamment un atome d'hydrogène ou un radical alkyle, le nombre total d'atomes de carbone dans R₁₈ et R₁₉ ne dépassant pas 10.
   Les polymères comprenant de tels motifs de formule (VII) peuvent comporter en outre des motifs dérivés de monomères non zwittérioniques tels que l'acrylate ou le méthacrylate de diméthyle ou de diéthylaminoéthyle, les acrylates ou méthacrylates d'alkyle, les acrylamides ou méthacrylamides ou l'acétate de vinyle.
   A titre d'exemple, on peut citer le copolymère méthacrylate de méthyle/méthacrylate de diméthylcarboxyméthylammonioéthyle tel que le produit commercialisé sous la dénomination DIAFORMER® Z301 par la Société SANDOZ.
(5) Les polymères dérivés du chitosane comportant des motifs monomères répondant aux formules suivantes : 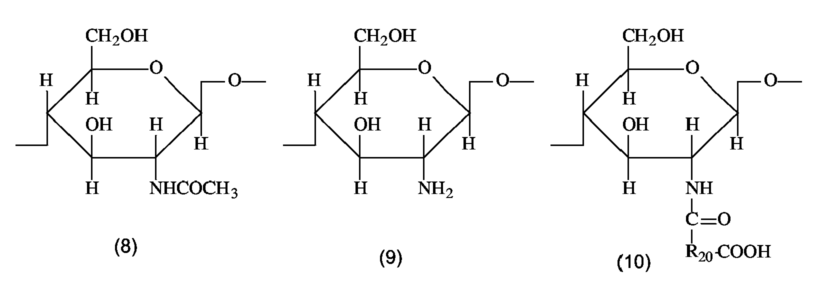 le motif de formule (8) étant présent dans des proportions comprises entre 0 et 30%, le motif de formule (9) dans des proportions comprises entre 5 et 50% et le motif de formule (10) dans des proportions comprises entre 30 et 90%, étant entendu que dans ce motif (10), R₂₀ représente un radical de formule (11) : 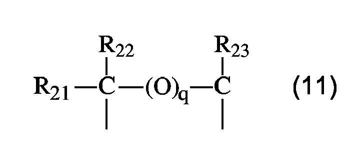 dans laquelle :
   si q = 0, alors R₂₁, R₂₂ et R₂₃, identiques ou différents, représentent chacun un atome d'hydrogène, un groupe méthyle, hydroxyle, acétoxy ou amino, un groupe monoalcoylamine ou dialcoylamine éventuellement interrompu par un ou plusieurs atomes d'azote et/ou éventuellement substitués par un ou plusieurs groupes amine, hydroxyle, carboxyle, alcoylthio ou sulfo, un groupe alcoylthio dont le groupe alcoyle porte un reste amino, l'un au moins des radicaux R₂₁, R₂₂ et R₂₃ étant dans ce cas un atome d'hydrogène ; ou si q = 1, alors R₂₁, R₂₂ et R₂₃ représentent chacun un atome d'hydrogène, ainsi que les sels formés par ces composés avec des bases ou des acides.
6) Les polymères obtenus par N-carboxyalkylation du chitosane, comme le N-carboxyméthylchitosane ou le N-carboxybutylchitosane commercialisé sous la dénomination EVALSAN® par la Société JAN DEKKER.
(7) Les polymères répondant à la formule générale (12) : 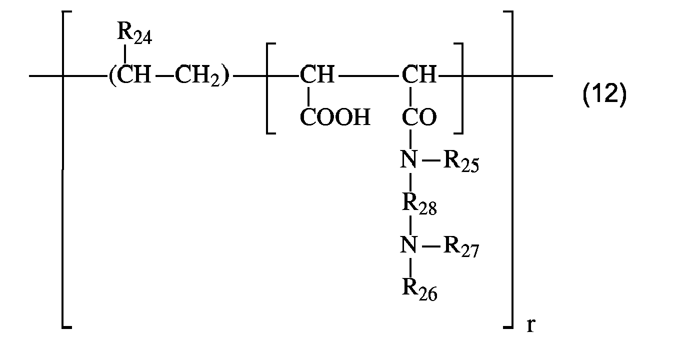 décrits notamment dans la demande de brevet FR 1 400 366, dans laquelle R₂₄ représente un atome d'hydrogène ou un radical CH₃O, CH₃CH₂O ou phényle, R₂₅ désigne un atome d'hydrogène ou un radical alkyle inférieur tel que méthyle ou éthyle, R₂₆ désigne un atome d'hydrogène ou un radical alkyle inférieur tel que méthyle ou éthyle, R₂₇ désigne un radical alkyle inférieur tel que méthyle ou éthyle ou un radical répondant à la formule :

   -R₂₈-N(R₂₂)₂,

   R₂₈ représentant un groupement -CH₂-CH₂-, -CH₂-CH₂-CH₂-,

   -CH2-CH(CH₃)- et R₂₂ ayant les significations mentionnées ci-dessus, ainsi que les homologues supérieurs de ces radicaux contenant jusqu'à 6 atomes de carbone.
(8) Les polymères amphotères du type -D-X-D-X- choisis parmi :
   (a) les polymères obtenus par action d'acide chloroacétique ou de chloroacétate de sodium sur les composés comportant au moins un motif de formule :

      (13) -D-X-D-X-D-

      où D désigne un radical : 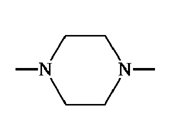 et X désigne le symbole E ou E', E ou E' identiques ou différents désignant un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote ou de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      (14) -D-X'-D-X'-
   où D désigne un radical 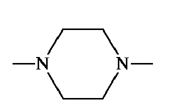 et X' désigne le symbole E ou E' et au moins une fois E', E ayant la signification indiquée ci-dessus et E' étant un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisée par réaction avec l'acide chloroacétique ou avec le chloroacétate de soude.
9) Les copolymères alkyl(C₁₋₅)vinyléther/anhydride maléique modifiés partiellement par semi-amidification avec une N,N-dialkylaminoalkylamine telle que la N,N-diméthylaminopropylamine, ou par semi-estérification avec une N,N-dialcanolamine. Ces copolymères peuvent également comporter d'autres comonomères vinyliques tels que le vinylcaprolactame.

Les polymères amphotères préférés selon l'invention sont ceux de la famille (3) décrite ci-dessus, tels que ceux dont la dénomination CTFA est « Octylacrylamide/acrylates/butylaminoéthyl-méthacrylate copolymer ». On peut citer à titre d'exemple les produits commercialisés sous les dénominations AMPHOMER®, AMPHOMER® LV 71 ou LOVOCRYL® 47 par la Société NATIONAL STARCH.

D'autres polymères amphotères préférés sont ceux de la famille (4), comme par exemple les copolymères de méthacrylate de méthyle et de méthacrylate de diméthylcarboxyméthylammonioéthyle, commercialisés par exemple sous la dénomination DIAFORMER® Z301 par la Société SANDOZ.

Les polymères anioniques ou amphotères peuvent, si nécessaire, être partiellement ou totalement neutralisés. Les agents de neutralisation sont par exemple la soude, la potasse, l'amino-2-méthylpropanol, la monoéthanolamine, la triéthanolamine ou la tri-isopropanolamine, les acides minéraux ou organiques tels que l'acide chlorhydrique ou l'acide citrique.

Les polymères non ioniques utilisables selon la présente invention sont choisis par exemple parmi :
- les homopolymères de vinylpyrrolidone,
- les copolymères de vinylpyrrolidone et d'acétate de vinyle,
- les polyalkyloxazolines telles que les polyéthyloxazolines proposées par la Société DOW CHEMICAL sous les dénominations PEOX® 50 000, PEOX® 200 000 et PEOX® 500 000,
- les homopolymères d'acétate de vinyle tels que le produit proposé sous le nom de APPRETAN® EM par la Société HOECHST ou le produit proposé sous le nom de RHODOPAS® A 012 par la Société RHONE POULENC ;
- les copolymères d'acétate de vinyle et d'esters acryliques tels que le produit proposé sous la dénomination RHODOPAS® AD 310 par RHONE POULENC,
- les copolymères d'acétate de vinyle et d'éthylène tels que le produit proposé sous le nom de APPRETAN® TV par la Société HOECHST,
- les copolymères d'acétate de vinyle et d'ester maléique par exemple de maléate de dibutyle tels que le produit proposé sous le nom de APPRETAN® MB EXTRA par la Société HOECHST,
- les copolymères d'éthylène et d'anhydride maléique,
- les homopolymères d'acrylates d'alkyle et les homopolymères de méthacrylates d'alkyle tels que le produit proposé sous la dénomination MICROPEARL® RQ 750 par la Société MATSUMOTO ou le produit proposé sous la dénomination LUHYDRAN® A 848 S par la Société BASF,
- les copolymères d'esters acryliques tels que par exemple les copolymères d'acrylates d'alkyle et de méthacrylates d'alkyle tels que les produits proposés par la Société ROHM & HAAS sous les dénominations PRIMAL AC-261 K et EUDRAGIT NE 30 D, par la Société BASF sous les dénominations ACRONAL® 601, LUHYDRAN® LR 8833
ou 8845, et par la Société HOECHST sous les dénominations APPRETAN® N 9213 ou N 9212,
- les copolymères d'acrylonitrile et d'un monomère non ionique choisi par exemple parmi le butadiène et les (méth)acrylates d'alkyle; on peut citer les produits proposés sous les dénominations NIPOL® LX 531 B par la Société NIPPON ZEON ou ceux proposés sous la dénomination CJ 0610 B par la Société ROHM & HAAS,
- les polyuréthanes tels que les produits proposés sous les dénominations ACRYSOL® RM 1020 OU ACRYSOL® RM 2020 par la Société ROHM & HAAS, les produits URAFLEX® XP 401 UZ, URAFLEX® XP et 402 UZ par la Société DSM RESINS,
- les copolymères d'acrylate d'alkyle et d'uréthane tels que le produit 8538-33 commercialisé par la Société NATIONAL STARCH,
- les polyamides tels que le produit ESTAPOR® LO 11 proposé par la Société RHONE POULENC,
- les gommes de guar non ioniques chimiquement modifiées
ou non modifiées,
- les alcools polyvinyliques, tels que le MOWIOL® 40-88 de la société HOECHST.

Les gommes de guar non ioniques non modifiées sont par exemple les produits vendus sous la dénomination VIDOGUM® GH 175 par la Société UNIPECTINE et sous la dénomination JAGUAR@ C par la Société MEYHALL. Les gommes de guar non ioniques modifiées utilisables selon l'invention sont de préférence modifiées par des groupements hydroxyalkyle en C₁-₈. On peut mentionner à titre d'exemple les groupements hydroxyméthyle, hydroxyéthyle, hydroxypropyle et hydroxybutyle.

Ces gommes de guar sont bien connues dans la technique et peuvent par exemple être préparées par réaction des oxydes d'alcènes correspondants, tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les dénominations commerciales JAGUAR@ HP8, JAGUAR@ HP60 et JAGUAR@ HP120, JAGUAR@ DC 293 et JAGUAR@ HP 105 par la Société MEYHALL, ou sous la dénomination GALACTASOL® 4H4FD2 par la Société AQUALON.

Le ou les agents moussants représentent de 0,01 à 20%, de préférence de 0,05 à 10%, en poids par rapport au poids total de composition cosmétique.

Parmi les alcanols utilisables selon la présente invention on peut citer les alcanols en C₁-C₆ tels que l'éthanol, le propanol, le butanol ou bien encore un mélange de ceux-ci. L'alcanol particulièrement préféré est l'éthanol.

Le ou les alcanols représentent généralement jusqu'à 20%, de préférence jusqu'à 15% en poids d'alcanol par rapport au poids total de la composition cosmétique.

La phase aqueuse selon l'invention peut également comprendre des agents nacrants.

Par agent nacrant, il faut comprendre au sens de la présente invention des particules irisées qui réfléchissent la lumière.

De tels agents nacrant peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité.

Dans le but d'améliorer les propriétés cosmétiques des fibres capillaires ou encore d'atténuer ou d'éviter leur dégradation, la composition utilisée selon l'invention peut également comprendre un ou plusieurs additifs cosmétiques.

Ce ou ces additifs sont généralement choisis parmi les agents protecteurs des fibres capillaires, les vitamines ou provitamines, les cires végétales, les céramides, les protéines, les parfums, les conservateurs, les séquestrants, les agents acidifiants, alcalinisants, ainsi que leurs mélanges.

Le ou les additifs cosmétiques ci-dessus représentent généralement de 0,001 à 20%, de préférence de 0,1 à 10%,en poids par rapport au poids total de la composition.

Les compositions comprises dans les sprays selon l'invention peuvent également comprendre des silicones.

Ces silicones peuvent être volatiles ou non, linéaires ou cycliques.

Les silicones utilisables conformément à l'invention sont en particulier des polyorganosiloxanes et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels, ainsi que leurs mélanges.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécane ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Les résines d'organopolysiloxanes utilisables conformément à l'invention sont des systèmes siloxaniques réticulés renfermant les unités

R'₂SiO_{2/2}, R'₃SiO_{1/2}, R'SiO_{3/2} et SiO_{4/2} dans lesquelles R' représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

Les silicones organomodifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut notamment citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (15) : 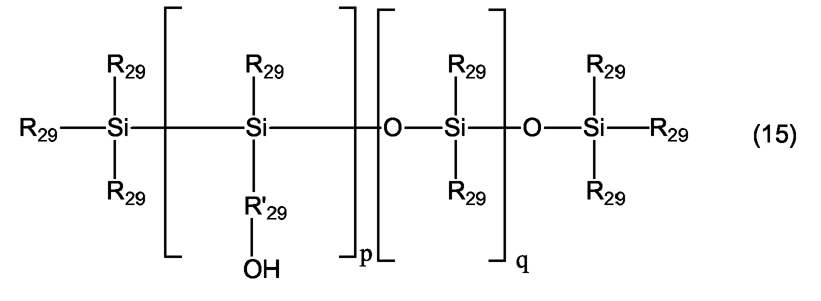 dans laquelle les radicaux R₂₉ identiques ou différents sont choisis parmi les radicaux méthyle et phényle ; au moins 60 % en mole des radicaux R₂₉ désignant méthyle ; le radical R'₂₉ est un chaînon alkylène divalent hydrocarboné en C₂-C₁₈ p est compris entre 1 et 30 inclus ; q est compris entre 1 et 150 inclus ;
   - des groupements acyloxyalkyle tels que par exemple les polyorganosiloxanes décrits dans le brevet US-A-4957732 et répondant à la formule (16) : 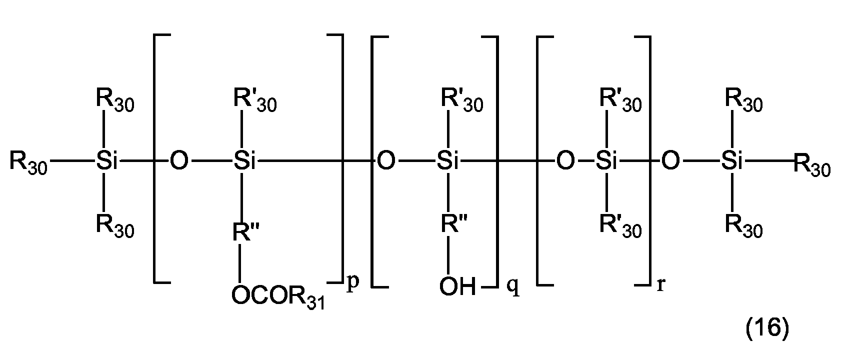
dans laquelle :
R₃₀ désigne un groupement méthyle, phényle, -OCOR₃, hydroxyle, un seul des radicaux R₃₀ par atome de silicium pouvant être OH ;
R'₃₀ désigne méthyle, phényle ; au moins 60 % en proportion molaire de l'ensemble des radicaux R₃₀ et R'₃₀ désignant méthyle ;
R₃₁ désigne alkyle ou alcényle en C₈-C₂₀ ;
R" désigne un radical alkylène hydrocarboné divalent, linéaire ou ramifié en C₂-C₁₈ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (16) peuvent contenir des groupements : 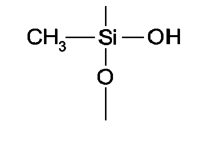
dans des proportions ne dépassant pas 15 % de la somme p + q + r.

- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Les polyorganosiloxanes particulièrement préférés conformément à l'invention sont polyorganosiloxanes comportant des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200.

Selon l'invention, toutes les silicones peuvent également être utilisées sous forme soluble, dispersée, micro dispersée, sous forme d'émulsions, de nanoémulsions ou de microémulsions.

En plus ou à la place des alcanols, la composition peut comprendre un ou plusieurs solvants cosmétiquement acceptables, choisis de préférence parmi les polyols tels que le glycérol, le propylène glycol, le pentanediol, et l'alcool benzylique.

Ce ou ces solvants cosmétiquement acceptables selon l'invention représentent de 0,001 à 20% du poids total de la composition, de préférence de 0,1 à 10% par rapport au poids total de la composition.

Les agents protecteurs des fibres capillaires peuvent être tout agent actif utile pour prévenir ou limiter les dégradations dues aux agressions physiques ou chimiques.

Ainsi, l'agent protecteur des fibres capillaires peut être choisi parmi les filtres UV organiques hydrosolubles, liposolubles, les agents antiradicalaires, les agents antioxydants, les vitamines, les provitamines ainsi que leurs mélanges.

Les filtres UV organiques (systèmes filtrant les radiations UV) sont notamment choisis parmi les filtres hydrosolubles ou liposolubles, siliconés ou non siliconés et les nanoparticules d'oxydes minéraux dont la surface a éventuellement été traitée pour la rendre hydrophile ou hydrophobe.

Les filtres UV organiques hydrosolubles peuvent être choisis parmi par exemple, l'acide para-aminobenzoïque et ses sels, l'acide anthranilique et ses sels, l'acide salicylique et ses sels, l'acide p-hydroxycinnamique et ses sels, les dérivés sulfoniques de benz-x-azole (benzothioazoles, benzimidazoles, benzoxazoles) et leurs sels, les dérivés sulfoniques de la benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole.

On peut également utiliser des polymères hydrophiles présentant, en outre et de par leur nature chimique, des propriétés de photoprotection contre le rayonnement UV. On peut citer les polymères comportant des groupements benzylidène camphre et/ou benzotriazole, substitués par des groupements sulfoniques ou ammonium quaternaires.

Comme filtres UV organiques liposolubles (ou lipophiles) convenant à une mise en oeuvre dans la présente invention, on peut notamment citer : les dérivés d'acide p-aminobenzoïque, tels que les esters ou amides d'acide p-aminobenzoïque ; les dérivés d'acide salicylique tels que les esters; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'ester éthylique d'acide urocanique ; les benzotriazoles ; les dérivés d'hydroxyphényltriazine ; les bis-résorcinol-dialkylaminotriazine ; et leurs mélanges.

Le filtre UV liposoluble (ou lipophile) selon l'invention est de préférence choisi parmi : l'octyl salicylate ; le 4-tertiobutyl 4'-méthoxydibenzoylméthane (PARSOL 1789 de GIVAUDAN); l'octocrylène ; le 4-méthoxy cinnamate de 2-éthylhexyl (PARSOL MCX) et le composé de formule (17) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, décrit dans la demande de brevet EP-A-0 392 883 :

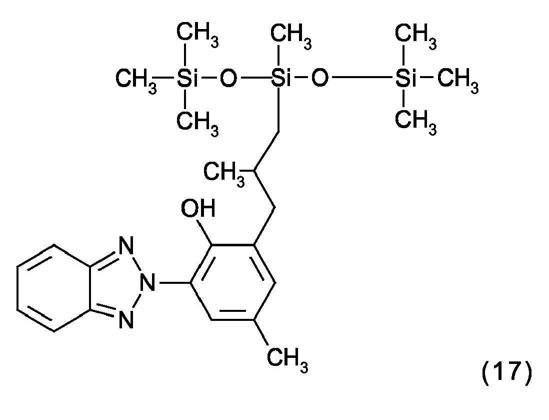

D'autres filtres UV particulièrement préférés selon l'invention sont les dérivés de benzophénones tels que l'UVINUL MS 40 (Acide 2-hydroxy 4-méthoxybenzophénone-5-sulfonique) et l'UVINUL M40 (2-hydroxy-4-méthoxybenzophénone) commercialisés par BASF, les dérivés de benzalmalonates tels que le PARSOL SLX (poly diméthyl/méthyl (3(4-(2,2-bis-éthoxycarbonylvinyl)-phénoxy)-propényl) siloxane) commercialisé par GIVAUDAN-ROURE, les dérivés de benzylidènecamphre tels que le MEXORYL SX (acide b-b'camphosulfonique [1-4 divinylbenzène]) fabriqué par la société CHIMEX, les dérivés de benzimidazole tels que l'EUSOLEX 232 (acide 2-phényl-benzimidazol-5-sulfonique) commercialisé par MERCK.

Le pH peut être ajusté à la valeur désirée au moyen d'agents acidifiants ou alcalinisants bien connus de l'état de la technique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (18) suivante : 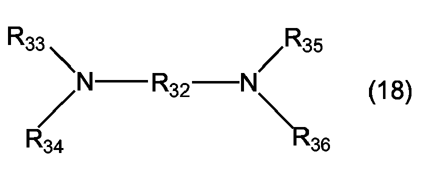 dans laquelle R₃₂ est un reste propylène éventuellement substitué par un groupement hydroxyle ou un radical alkyle en C₁-C₄ ; R₃₃, R₃₄, R₃₅ et R₃₆, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Comme indiqué précédemment, dispositif pressurisé, notamment aérosol de coiffage et/ou de soin capillaire selon l'invention comprend un dispositif de restitution sous pression de la composition.

Le dispositif de restitution sous pression de la composition, qui forme une partie du récipient aérosol, est généralement constitué d'une valve de distribution commandée par un bouton poussoir, lui-même comprenant un diffuseur par lequel la composition aérosol est restituée sous forme d'une mousse.

De manière préférentielle, la composition est restituée sous forme d'une mousse ayant une densité par rapport à l'eau allant jusqu'à 0,1.

La phase organique de la composition contenue dans le dispositif pressurisé objet de l'invention comprend également un agent propulseur choisi parmi les hydrocarbures tels que le n-butane, le propane, l'isobutane, le pentane, les hydrocarbures chlorés et/ou fluorés et leurs mélanges.

Parmi les hydrocarbures halogénés préférés, on peut citer le 1,1-difluoroéthane.

La phase organique de la composition étant pressurisée dans le récipient, elle se présente généralement sous forme de liquide dans celui-ci.

De préférence, l'hydrocarbure choisi est l'isobutane.

La pression à 20°C du dispositif pressurisé pourra notamment être régulée selon la nature de l'hydrocarbure ou des hydrocarbures employés.

Le ou les agents propulseurs représentent généralement jusqu'à 25%, de préférence de 2 à 15% et encore plus préférentiellement de 5 à 12% en poids par rapport au poids total de la composition telle que décrite ci-dessus.

Un mode de réalisation du dispositif pressurisé de l'invention est représenté sur les dessins annexés et est décrit ci-après à titre d'exemple purement illustratif et nullement limitatif.
La figure 1A représente une vue axiale d'un récipient aérosol conforme à l'invention, montré en position ouverte.
La figure 1 B représente une vue de dessous du récipient.
La figure 1C représente une vue en coupe de la cupule de renforcement.
La figure 2A représente une vue axiale d'un autre mode de réalisation du récipient comprenant un fond de forme concave.
La figure 2B représente une vue du dessous du fond de forme concave.
La figure 2C représente une vue isométrique du fond représenté aux figures 2A et 2B.

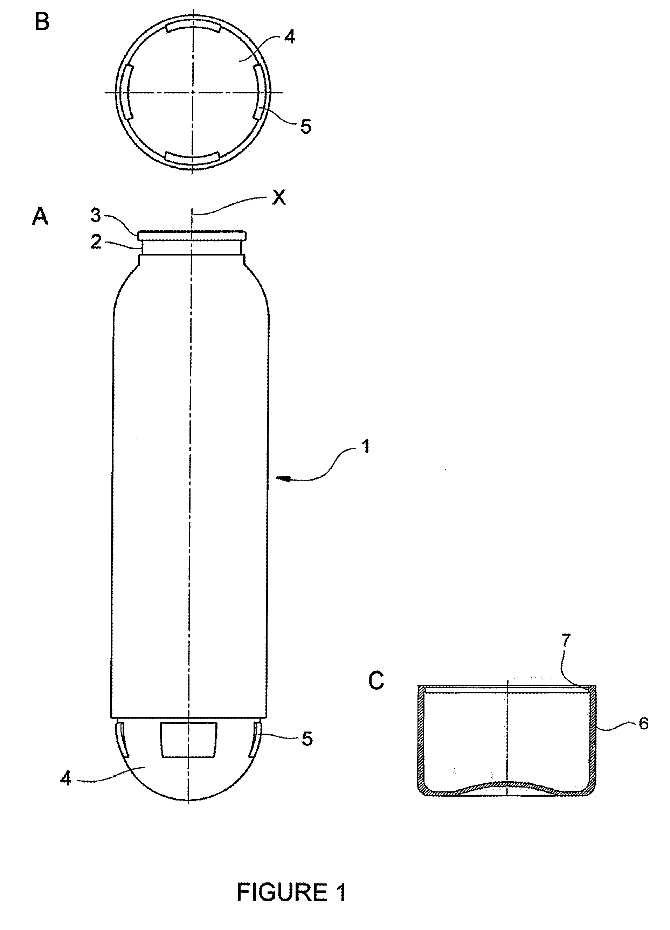

Sur la figure 1A, on a représenté un récipient désigné dans son ensemble par la référence 1.

De préférence, le récipient comprend au moins une portion cylindrique d'axe longitudinal X, portant un col ouvert 2. Il est bien entendu que le récipient peut avoir une forme légèrement aplatie ou étirée selon l'axe X, ce qui peut être intéressant pour des raisons d'esthétique.

De manière préférentielle, le récipient du dispositif selon l'invention est cylindrique sur toute sa hauteur.

La forme cylindrique de ce récipient permet notamment de rendre le dispositif pressurisé selon l'invention aisément reconnaissable par les consommateurs comme dispositif pressurisé de coiffage.

De plus, lorsque plusieurs récipients sont conditionnés ou disposés ensemble, ceux de forme cylindrique occupent moins d'espace que ceux de forme sphérique, ce qui permet un gain de place lors de leur conditionnement ou de leur disposition sur des présentoirs.

L'ouverture formée à l'extrémité libre du col 2 présente un bourrelet 3 sur lequel est généralement fixée une coupelle porte-valve non représentée ici.

Sur la figure 1 B, on a représenté le récipient qui comprend une cupule de renforcement 6 disposée sur le fond 4.

Le fond 4 peut être de forme hémisphérique convexe et la cupule de renforcement 6 peut être munie d'une collerette 7 permettant l'accrochage de la cupule sur le fond 4.

Le fond hémisphérique peut comprendre des ergots 5 qui vont coopérer avec la collerette pour permettre le maintien de la cupule 6 avec le fond 4 du récipient 1 (figure 1 C).

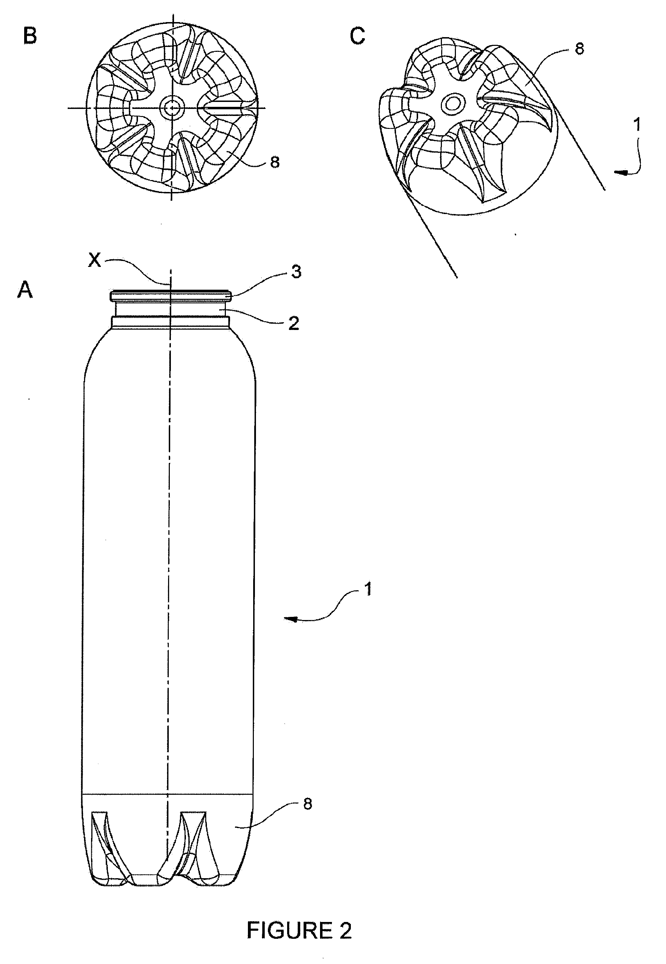

Les figures 2A, 2B et 2C représentent un autre mode de réalisation du dispositif pressurisé selon l'invention comprenant un récipient ayant un fond 8 concave.

De manière préférentielle, le récipient comprend un fond ayant une concavité.

Selon un autre mode de réalisation, le récipient comprend un fond ayant une pluralité de concavités.

Selon un autre mode de réalisation, le récipient selon l'invention comprend un fond plat.

De manière préférentielle, le récipient comprend une cupule de renforcement disposée sur le fond.

Préférentiellement, l'épaisseur de la paroi du récipient hors col et hors fond est comprise entre 0,45 et 1 mm.

La résistance du récipient selon l'invention à la pression d'épreuve et sa tenue au vide ont notamment été déterminées.

On entend par résistance à la pression d'épreuve la pression à laquelle le récipient vide peut être soumis pendant 25 secondes à 20°C sans qu'une fuite ne se produise ou que le récipient ne présente des déformations visibles ou permanentes.

On entend par tenue au vide la dépression à laquelle le récipient peut être soumis pendant 25 secondes à 20°C sans qu'une entrée d'air ne se produise ou que le récipient ne présente des déformations visibles ou permanentes.

Le récipient selon l'invention a montré une résistance à une pression d'épreuve supérieure à 20 bars, et une tenue au vide supérieure à 0,6 bar. Le récipient selon l'invention est généralement obtenu par étirement-soufflage selon des techniques bien connues de l'homme du métier.

L'invention a également pour objet une composition pour le coiffage des fibres capillaires conditionnée en dispositif pressurisé tel que décrit ci-dessus.

La présente invention est illustrée par l'exemple suivant:

### EXEMPLE

On réalise une mousse aérosol pour le coiffage et/ou le soin des cheveux en pressurisant dans un récipient en polyéthylène naphtalate et polyéthylène téréphtalate (90:10) une composition cosmétique biphasée qui comprend, en poids par rapport au poids total de la composition :

| | |
|---|---|
| Polyquaternium-30 : | 0,55% |
| Polyquaternium-4 : | 0,5% |
| Laureth-4 : | 0,05% |
| Copolymer VC 713 (ISP) : | 0,7% M.A |
| Mowiol 40-88 (Hoechst) : | 0,1% M.A |
| Ethanol : | 8% |
| Parfum: | 0,1% |
| Eau : | 80% |
| Isobutane : | 10% |

### M.A : Matière Active

Le produit aérosol ainsi obtenu est restitué sous forme d'une mousse pouvant être aisément appliquée sur les fibres capillaires pour leur coiffage.

## Revendications

1. Dispositif pressurisé, notamment aérosol, comprenant :
a) un récipient dont la paroi est formée au moins en partie d'un mélange d'au moins un polyéthylène naphtalate et d'au moins un polyéthylène téréphtalate,
b) une composition cosmétique comprenant :
- une phase aqueuse comprenant au moins un agent moussant ; et
- une phase organique comprenant au moins un agent propulseur choisi parmi les hydrocarbures halogénés ou non et leurs mélanges,
ledit dispositif étant résistant à la température.

2. Dispositif selon la revendication 1, **caractérisé en ce que** le récipient a) est transparent ou translucide.

3. Dispositif selon l'une quelconque des revendications 1 ou 2, **caractérisé en ce que** la composition b) est transparente ou translucide.

4. Dispositif selon l'une quelconque des revendications précédentes, **caractérisé en ce que** le récipient a) comporte au moins une portion cylindrique.

5. Dispositif selon la revendication 4, **caractérisé en ce que** le récipient a) est cylindrique sur toute sa hauteur.

6. Dispositif selon l'une quelconque des revendications précédentes, **caractérisé en ce que** le récipient a) comprend un fond concave.

7. Dispositif selon la revendication 6, **caractérisé en ce que** le récipient a) comprend un fond ayant une concavité.

8. Dispositif selon la revendication 6, **caractérisé en ce que** le récipient a) comprend un fond ayant une pluralité de concavités

9. Dispositif selon l'une quelconque des revendications 1 à 5, **caractérisé en ce que** le récipient comprend un fond plat.

10. Dispositif selon l'une quelconque des revendications précédentes, **caractérisé en ce que** le récipient comprend une cupule de renforcement disposée sur le fond.

11. Dispositif selon l'une quelconque des revendications précédentes, **caractérisé en ce que** le récipient comprend, en poids par rapport au poids total de polymère, de 60% à 95%, de préférence 90% de polyéthylène naphtalate et de 5% à 40%, de préférence 10% de polyéthylène téréphtalate.

12. Dispositif selon l'une quelconque des revendications précédentes, **caractérisé en ce que** le ou les agents moussants sont choisis parmi les tensio-actifs cationiques, anioniques, non-ioniques, amphotères ou zwittérioniques et les polymères cationiques, anioniques, non-ioniques, amphotères ou zwittérioniques, ainsi que leurs mélanges.

13. Dispositif selon l'une quelconque des revendications précédentes, **caractérisé en ce que** le ou les agents moussants représentent de 0,01 à 20%, de préférence de 0,05 à 10%, en poids par rapport au poids total de la composition cosmétique.

14. Dispositif selon l'une quelconque des revendications précédentes, **caractérisé en ce que** la composition cosmétique comprend au moins un alcanol choisi parmi les alcanols en C₁- C₆.

15. Dispositif selon l'une quelconque des revendications précédentes, **caractérisé en ce que** la composition cosmétique comprend jusqu'à 20%, de préférence jusqu'à 15% en poids d'alcanol par rapport au poids total de la composition.

16. Dispositif selon l'une quelconque des revendications précédentes, **caractérisé en ce que** la composition cosmétique comprend au moins additif cosmétique choisi parmi les agents protecteurs des fibres capillaires, les vitamines ou provitamines, les cires végétales, les céramides, les protéines, les parfums, les conservateurs, les séquestrants, les agents acidifiants, les agents alcalinisants et leurs mélanges.

17. Dispositif selon l'une quelconque des revendications précédentes, **caractérisé en ce que** le ou les additifs cosmétiques sont présents dans la composition cosmétique à raison de 0,001% à 20%, de préférence de 0,1 à 10% en poids par rapport au poids total de la composition.

18. Dispositif selon l'une quelconque des revendications précédentes, **caractérisé en ce que** la composition cosmétique contient au moins une silicone volatile ou non, linéaire ou cyclique.

19. Dispositif selon la revendication 18 **caractérisé en ce qu'**au moins une silicone est choisie parmi les polyorganosiloxanes

20. Dispositif selon la revendication 19, **caractérisé en ce que** les polyorganosiloxanes sont choisis parmi les polyalkylsiloxanes, les polyarylsiloxanes, les polyalkylarylsiloxanes, les polyorganosiloxanes modifiés par des groupements organofonctionnels, ainsi que leurs mélanges.

21. Dispositif selon l'une quelconque des revendications 18 à 20, **caractérisé en ce que** les silicones sont également utilisées sous forme soluble, dispersée, micro dispersée, d'émulsions, de nanoémulsions ou de micromulsions.

22. Dispositif selon l'une quelconque des revendications précédentes, **caractérisé en ce que** la composition cosmétique comprend au moins un agent protecteur des fibres capillaires choisi parmi les filtres UV organiques hydrosolubles ou liposolubles, les agents antiradicalaires, les agents antioxydants, les vitamines, les provitamines ainsi que leurs mélanges.

23. Dispositif selon l'une quelconque des revendications précédentes, **caractérisé en ce que** la composition cosmétique comprend un ou plusieurs solvants cosmétiquement acceptables choisis parmi les polyols tels que le glycérol, le propylène glycol, le pentanediol et l'alcool benzylique.

24. Dispositif selon la revendication 23, **caractérisé en ce que** ce ou ces solvants cosmétiquement acceptables représentent de 0,001 à 20%, de préférence de 0,1 à 10% par rapport au poids total de la composition cosmétique.

25. Dispositif selon l'une quelconque des revendications précédentes, **caractérisé en ce que** l'agent propulseur est choisi parmi les hydrocarbures tels que le n-butane, le propane, l'isobutane, le pentane, les hydrocarbures chlorés et/ou fluorés et leurs mélanges.

26. Dispositif selon l'une quelconque des revendications précédentes, **caractérisé en ce que** l'agent propulseur représente jusqu'à 30%, de préférence de 2 à 15% et encore plus préférentiellement de 5 à 12% en poids par rapport au poids total de la composition cosmétique.

27. Dispositif selon l'une quelconque des revendications précédentes, **caractérisé en ce que** l'agent propulseur choisi est l'isobutane.

28. Dispositif selon l'une quelconque des revendications précédentes, **caractérisé en ce que** la composition est restituée sous forme d'une mousse ayant une densité par rapport à l'eau allant jusqu'à 0,1.

29. Composition cosmétique comprenant :
- une phase aqueuse comprenant au moins un agent moussant ; et
- une phase organique comprenant au moins un agent propulseur choisi parmi les hydrocarbures halogénés ou non et leurs mélanges,
**caractérisée en ce qu'**elle est conditionnée en dispositif pressurisé, notamment aérosol dont la paroi est formée au moins en partie d'un mélange d'au moins un polyéthylène naphtalate et d'au moins un polyéthylène téréphtalate, ledit dispositif étant résistant à la température.

30. Composition cosmétique selon la revendication 29, **caractérisée en ce que** le récipient est transparent ou translucide.

31. Composition cosmétique selon l'une quelconque des revendications 29 ou 30, **caractérisée en ce que** la composition est transparente ou translucide.

32. Composition cosmétique selon l'une quelconque des revendications 29 à 31, **caractérisée en ce que** le récipient comporte au moins une portion cylindrique.

33. Composition cosmétique selon l'une quelconque des revendications 29 à 32, **caractérisée en ce que** le récipient est cylindrique sur toute sa hauteur.

34. Composition cosmétique selon l'une quelconque des revendications 29 à 33, **caractérisée en ce que** le récipient comprend un fond concave.

35. Composition cosmétique selon la revendication 34, **caractérisée en ce que** le récipient comprend un fond ayant une concavité.

36. Composition cosmétique selon la revendication 34, **caractérisée en ce que** le récipient comprend un fond ayant une pluralité de concavités

37. Composition cosmétique selon l'une quelconque des revendications 29 à 34, **caractérisée en ce que** le récipient comprend un fond plat.

38. Composition cosmétique selon l'une quelconque des revendications 29 à 37, **caractérisée en ce que** le récipient comprend une cupule de renforcement disposée sur le fond.

39. Composition cosmétique selon l'une quelconque des revendications 29 à 38, **caractérisée en ce que** le récipient comprend, en poids par rapport au poids total de polymère, de 60% à 95%, de préférence 90% de polyéthylène naphtalate et de 5% à 40%, de préférence 10% de polyéthylène téréphtalate.

40. Composition cosmétique selon l'une quelconque des revendications 29 à 39, **caractérisée en ce que** le ou les agents moussants sont choisis parmi les tensio-actifs cationiques, anioniques, non-ioniques, amphotères ou zwittérioniques et les polymères cationiques, anioniques, non-ioniques, amphotères ou zwittérioniques, ainsi que leurs mélanges.

41. Composition cosmétique selon l'une quelconque des revendications 29 à 4, **caractérisée en ce que** le ou les agents moussants représentent de 0,01 à 20%, de préférence de 0,05 à 10%, en poids par rapport au poids total de la composition cosmétique.

42. Composition cosmétique selon l'une quelconque des revendications 29 à 41, **caractérisée en ce que** la composition cosmétique comprend au moins un alcanol choisi parmi les alcanols en C₁- C₆.

43. Composition cosmétique selon l'une quelconque des revendications 29 à 42, **caractérisée en ce qu'**elle comprend jusqu'à 20%, de préférence jusqu'à 15% en poids d'alcanol par rapport au poids total de la composition.

44. Composition cosmétique selon l'une quelconque des revendications 29 à 43, **caractérisée en ce qu'**elle comprend au moins additif cosmétique choisi parmi les agents protecteurs des fibres capillaires, les vitamines ou provitamines, les cires végétales, les céramides, les protéines, les parfums, les conservateurs, les séquestrants, les agents acidifiants, les agents alcalinisants et leurs mélanges.

45. Composition cosmétique selon l'une quelconque des revendications 29 à 44, **caractérisée en ce que** le ou les additifs cosmétiques sont présents dans la composition cosmétique à raison de 0,001% à 20%, de préférence de 0,1 à 10% en poids par rapport au poids total de la composition.

46. Composition cosmétique selon l'une quelconque des revendications 29 à 45, **caractérisée en ce que** la composition cosmétique contient au moins une silicone volatile ou non, linéaire ou cyclique.

47. Composition cosmétique selon la revendication 46 **caractérisée en ce qu'**au moins une silicone est choisie parmi les polyorganosiloxanes

48. Composition cosmétique selon la revendication 47, **caractérisée en ce que** les polyorganosiloxanes sont choisis parmi les polyalkylsiloxanes, les polyarylsiloxanes, les polyalkylarylsiloxanes, les polyorganosiloxanes modifiés par des groupements organofonctionnels, ainsi que leurs mélanges.

49. Composition cosmétique selon l'une quelconque des revendications 46 à 48, **caractérisée en ce que** les silicones sont également utilisées sous forme soluble, dispersée, micro dispersée, d'émulsions, de nanoémulsions ou de micromulsions.

50. Composition cosmétique selon l'une quelconque des revendications 29 à 49, **caractérisée en ce qu'**elle comprend au moins un agent protecteur des fibres capillaires choisi parmi les filtres UV organiques hydrosolubles ou liposolubles, les agents antiradicalaires, les agents antioxydants, les vitamines, les provitamines ainsi que leurs mélanges.

51. Composition cosmétique selon l'une quelconque des revendications 29 à 50, **caractérisée en ce qu'**elle comprend un ou plusieurs solvants cosmétiquement acceptables choisis parmi les polyols tels que le glycérol, le propylène glycol, le pentanediol et l'alcool benzylique.

52. composition cosmétique selon la revendication 51, **caractérisée en ce que** ce ou ces solvants cosmétiquement acceptables représentent de 0,001 à 20%, de préférence de 0,1 à 10% par rapport au poids total de la composition cosmétique.

53. Composition cosmétique selon l'une quelconque des revendications 29 à 52, **caractérisée en ce que** l'agent propulseur est choisi parmi les hydrocarbures tels que le n-butane, le propane, l'isobutane, le pentane, les hydrocarbures chlorés et/ou fluorés et leurs mélanges.

54. Composition cosmétique selon l'une quelconque des revendications 29 à 53, **caractérisée en ce que** l'agent propulseur représente jusqu'à 30%, de préférence de 2 à 15% et encore plus préférentiellement de 5 à 12% en poids par rapport au poids total de la composition cosmétique.

55. Composition cosmétique selon l'une quelconque des revendications 29 à 54, **caractérisée en ce que** l'agent propulseur choisi est l'isobutane.

56. Composition cosmétique selon l'une quelconque des revendications 29 à 55, **caractérisée en ce qu'**elle est restituée sous forme d'une mousse ayant une densité par rapport à l'eau allant jusqu'à 0,1.
